# EUROPEAN PATENT APPLICATION

(11) **EP 3 599 233 A1**
(43) Date of publication of application: **29.01.2020**
(21) Application number: 19184230.1
(22) Date of filing: 03.07.2019
(51) Int. Cl.: C07D 209/46, C08G 64/12

(54) **METHODS OF PURIFICATION OF 2-ARYL-3,3-BIS(4-HYDROXYARYL)PHTHALIMIDINES, AND POLYMERS DERIVED THEREFROM**

(30) Priority: 23.07.2018 EP 18185057
(71) Applicant: SABIC Global Technologies B.V., 4612 PX Bergen op Zoom (NL)
(72) Inventor: Sandesh Shivajirao, Deshmukh, 562125 Bangalore, Karnataka (IN); Konda, Shivakumar, 562125 Bangalore, Karnataka (IN); Sreeremagiri, Sivakumar, 562125 Bangalore, Karnataka (IN); Periyasamy, Sivakumar, 562125 Bangalore, Karnataka (IN); Gautam, Ravi, 562125 Bangalore, Karnataka (IN); Namala, Srinivasan, 500033 Telangana (IN); Balakrishnan, Ganesan, 500055 Telangana (IN)
(74) Representative: Balder IP Law, S.L.

(57) **Abstract**

A method for the purification of a 2-aryl-3,3-bis(hydroxyaryl)phthalimidine of formula (I), the method comprising heating a reaction mixture comprising a phenolphthalein compound of formula (II) and a primary arylamine of formula (III) in the presence of an acid catalyst to form a reaction mixture; removing water from the reaction mixture; quenching the reaction mixture with an aqueous alkali solution to form a quenched reaction mixture; extracting the quenched reaction mixture with an aminoaryl compound of formula (IV) to form an organic layer and an extracted aqueous layer comprising a crude 2-aryl-3,3-bis(hydroxyaryl)phthalimidine compound; contacting the extracted aqueous layer with carbon to form a semi-purified 2-aryl-3,3-bis(hydroxyaryl)phthalimidine compound; and mixing the semi-purified 2-aryl-3,3-bis(hydroxyaryl)phthalimidine compound with a solution comprising an alcohol and an acid to form a purified 2-aryl-3,3-bis(hydroxyaryl)phthalimidine of formula (I).

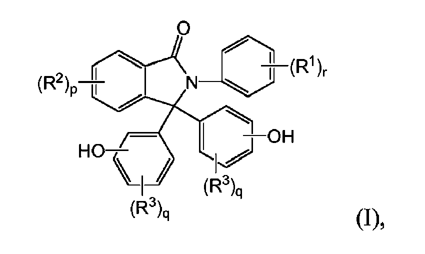

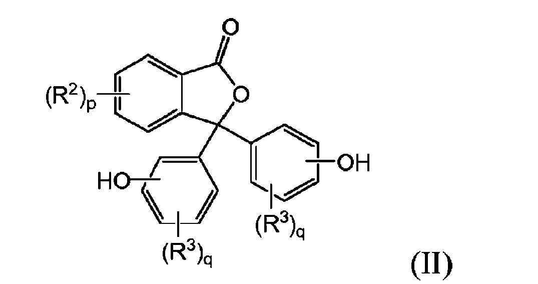

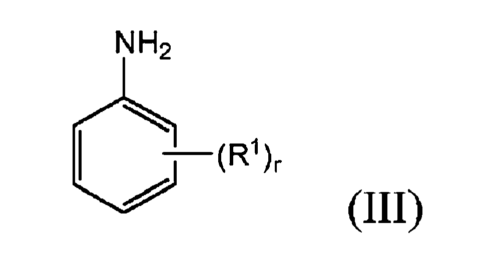

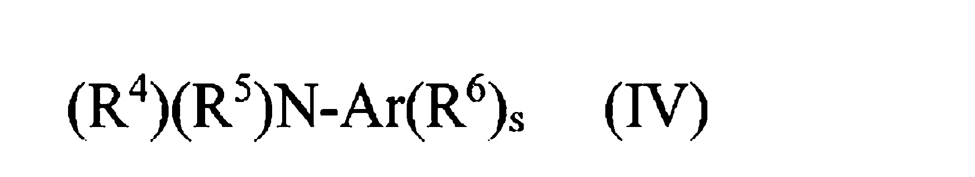

## Description

### BACKGROUND

Phenolphthalein derivatives such as 2-phenyl-3,3-bis(4-hydroxyphenyl)phthalimidine (also known as N-phenyl phenolphthalein bisphenol (PPPBP) or 3,3-bis(4-hydroxyphenyl)-2-phenylisoindolin-1-one)) have been used as aromatic dihydroxy compound monomers to prepare polycarbonate resins as well as polyarylate resins. Phenolphthalein derivatives can be difficult to make and isolate with sufficient purity for use in polymer synthesis. Currently available methods to make and isolate phenolphthalein derivatives are lengthy and resource intensive. Purification of crude PPPBP is usually also conducted in a batch process, for example as described in U.S. Patent 7,135,577, U.S. Patent 7,563,817, and U.S. Patent 7,884,220. The total process can involve eight, ten, or more manual interventions, which can become particular problematic on the industrial scale.

In addition, the isolated phenolphthalein derivatives are usually in the form of a fine powder having a mean particle size of less than 2 micrometers. The small particle size can cause problems during the preparation of polymers and/or copolymers from the phenolphthalein derivatives. Among these problems is that these small particles generate airborne dust, which can cause environmental, health and safety issues, such as the risk of dust explosion in an area where the phenolphthalein derivative are stored or handled, such as in a chemical plant environment. Another problem is that the fine nature of the particles causes a "slug flow" behavior during operations such as charging the phenolphthalein derivative powders into a polymerization reactor, which in turn may lead to an erratic reaction, or to a reaction that is difficult to control. Further, the "slug flow" behavior also causes problems during general transfer of the fine powder from one container to another, particularly on a large scale, such as in a plant environment.

Accordingly, there remains a need for improved methods for the purification of phenolphthalein derivatives. Furthermore, it would desirable if the purification methods could also provide a purified product having an increased mean particle size.

### BRIEF SUMMARY

Provided is a method for the purification of a 2-aryl-3,3-bis(hydroxyaryl)phthalimidine of formula (I), comprising heating a reaction mixture comprising a phenolphthalein compound of formula (II) and a primary arylamine of formula (III) in the presence of an acid catalyst to form a reaction mixture; removing water from the reaction mixture, preferably by removing an arylamine-water azeotrope from the reaction mixture; quenching the reaction mixture with an aqueous alkali solution to form a quenched reaction mixture; extracting the quenched reaction mixture with an aminoaryl compound of formula (IV) to form an organic layer and an extracted aqueous layer comprising a crude 2-aryl-3,3-bis(hydroxyaryl)phthalimidine compound; contacting the extracted aqueous layer with carbon to form a semi-purified 2-aryl-3,3-bis(hydroxyaryl)phthalimidine compound, and optionally further comprising repeating the contacting with the carbon; and mixing the semi-purified 2-aryl-3,3-bis(hydroxyaryl)phthalimidine compound with a solution comprising an alcohol and an acid to form a purified 2-aryl-3,3-bis(hydroxyaryl)phthalimidine of formula (I); wherein in formulas (I), (II), (III), and (IV), each occurrence of R¹ is independently a phenyl or a C₁₋₂₅ hydrocarbyl, preferably a phenyl or a C₁₋₆ alkyl, more preferably a C₁₋₃ alkyl, each occurrence of R² and R³ is independently a C₁₋₂₅ hydrocarbyl or halogen, preferably a C₁₋₆ alkyl, more preferably a C₁₋₃ alkyl, each R⁴ and R⁵ is independently a hydrogen or C₁₋₂₅ hydrocarbyl, preferably a hydrogen or C₁₋₆ alkyl, more preferably a hydrogen, Ar is a C₆₋₁₂ aromatic ring optionally comprising up to three heteroatoms in the ring, preferably a C₆ or a C₁₂ aromatic ring, each R⁶ is independently a halogen, nitro, cyano, or C₁₋₂₅ hydrocarbyl, preferably a C₁₋₆ alkyl, more preferably a C₁₋₃ alkyl, and r, p, q, and s are each independently 0 to 4, more preferably 0 or 1, preferably 0.

Also provided is a polycarbonate composition, wherein a total content of aminophenol impurity and phenolphthalein impurity is less than 0.2 weight percent, preferably less than 0.1 weight percent, more preferably less than 0.025 weight percent, based on the total weight of the polycarbonate.

Also provided is a 2-aryl-3,3-bis(4-hydroxyaryl)phthalimidine composition, comprising the purified 2-aryl-3,3-bis(hydroxyaryl)phthalimidine compound, wherein a total content of aminophenol impurity and phenolphthalein impurity is less than 1 weight percent, preferably less than 0.05 weight percent, more preferably less than 0.03 weight percent, based on the total weight of the composition.

The above described and other features are exemplified by the following figures and detailed description.

### BRIEF DESCRIPTION OF THE DRAWING

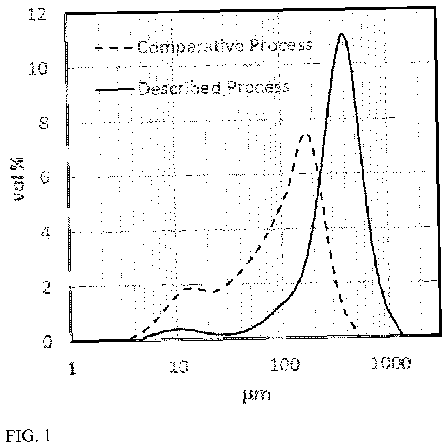

FIG. 1 is a graph of volume (%) versus particle size (micron, µm) comparing the particle size distribution from the comparative process to the process according to an aspect.

### DETAILED DESCRIPTION

As mentioned previously, the present disclosure is directed to the purification of 2-aryl-3,3-bis(4-hydroxyaryl)phthalimidines to provide an isolated product having lower levels of trace impurities, such as aminophenols and phenolphthalein, and an increased mean particle size. For example, the purified phthalimidine compound can have less than 1 weight percent (wt%) of total impurities and an average particle size of greater than 150 micrometers (µm). The 2-aryl-3,3-bis(4-hydroxyaryl)phthalimidines purified in accordance with these methods can be used in the manufacture of polycarbonates and other polymers. The method includes heating a reaction mixture including a phenolphthalein compound and a primary aryl amine in the presence of an acid catalyst to form a reaction mixture. The method further includes removing water from the reaction mixture, quenching the reaction mixture with an aqueous alkali solution to form a quenched reaction mixture, and extracting the quenched mixture with an aminoaryl compound. The method further includes contacting an extracted aqueous layer with carbon to form a semi-purified 2-aryl-3,3-bis(hydroxyaryl)phthalimidine compound and further purifying the product for example by mixing with a methanol and acid solution. The methods described to purify 2-aryl-3,3-bis(4-hydroxyaryl)phthalimidines can improve the overall manufacturing process and allow for a continuous process, reduce equipment usage and labor, and also simplify further downstream processing.

The 2-aryl-3,3-bis(4-hydroxyaryl)phthalimidine compounds are of formula (I) 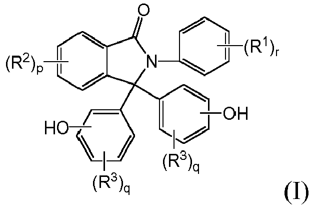 wherein each R¹ is independently a phenyl or a C₁₋₂₅ hydrocarbyl, each R² and R³ is independently a C₁₋₂₅ hydrocarbyl or halogen, and r, p, and q are each independently 0-4. For example, each R¹ can be independently a phenyl or a C₁₋₆ alkyl, each R² and R³ is independently a C₁₋₆ alkyl, and r, p, and q are each independently 0 or 1. For example, R¹ can be phenyl or a C₁₋₃ alkyl group, r and p are each independently 0-4. For example, r and p each can be independently 0 or 1. For example, each q can be 0, and R² can be a C₁₋₃ alkyl group or a halogen. In some aspects, r, p, and q are each 0.

The 2-aryl-3,3-bis(4-hydroxyaryl)phthalimidine compound can be of formula (IA) 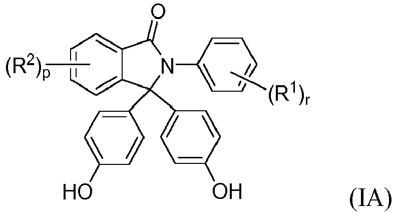 wherein R¹ is a C₁₋₃ alkyl, R² is a C₁₋₃ alkyl or a halogen, p is 0 or 1, and r is 0 or 1. For example, each of p and r can be zero. When each of p, q, and r is 0 in formulas (I) and (IA), the 2-aryl-3,3-bis(4-hydroxyaryl)phthalimidine compound is a 2-phenyl-3,3-bis(4-hydroxyphenyl)-2-phthalimidine compound of formula (IB)

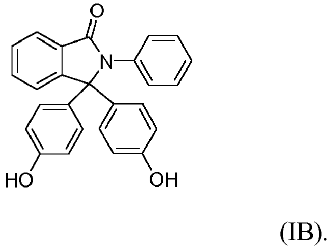

The 2-aryl-3,3-bis(4-hydroxyaryl)phthalimidine compound is prepared by heating a reaction mixture including a phenolphthalein compound of formula (II) 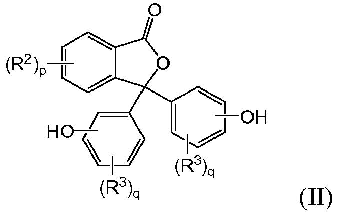 and a primary arylamine of formula (III) 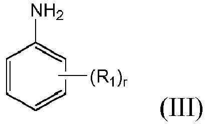 in the presence of an acid catalyst to form a reaction mixture. In formulas (II) and (III), R¹, R², R³, and p, q, and r are each as defined in formula (I). Exemplary primary arylamines include aniline.

Exemplary acid catalysts include mineral acids. The mineral acids can be present in a fluid phase, for example, in a gaseous phase or in a liquid phase or in a combination of the gaseous and liquid phases. Non-limiting examples of mineral acids include hydrogen chloride liquid, hydrogen chloride gas, sulfuric acid, nitric acid, and the like.

The acid catalyst can be present at a concentration of 0.5 to 1.5 molar equivalents of the phenolphthalein compound of formula (II). For example, the acid catalyst can be present at a concentration of 0.75 to 1.5, or 0.75 to 1.25, or 0.8 to 1.2 molar equivalents of the phenolphthalein (II).

The mineral acid can form an amine salt from the reaction of the mineral acid with an amine, and the amine salt can function as a catalyst in the formation of phthalimidine (I). Exemplary amines for forming the acid catalysts include primary, secondary, and tertiary amines having any combination of aliphatic and aromatic groups bonded to the amine nitrogen. For example, the primary arylamine of formula (III) can be used to form the amine salt.

The heating can be at a temperature of 135 to 180°C. For example, the heating can be at a temperature of 155 to 175°C. The heating can be for 5 to 20 hours. For example, the heating can be for 8 to 15 hours.

The method further includes removing water from the reaction mixture. The water can be continuously removed from the reaction mixture. Removing water can be, for example by heating the reaction mixture above 120°C, or removing water as an azeotrope with the primary arylamine of formula (III), e.g., aniline.

The reaction mixture is quenched by reaction with an aqueous alkali solution to form a quenched reaction mixture. The aqueous alkali solution can include an aqueous solution of an alkali metal hydroxide or an alkaline earth metal hydroxide. For example, the aqueous alkali solution can include sodium hydroxide.

The aqueous alkali solution can be present at a concentration of 1.5 to 3.0 molar equivalents of the phenolphthalein compound of formula (II). For example, the aqueous alkali solution can be present at a concentration of 1.5 to 2.5, or 1.75 to 2.75, or 2.0 to 3.0 molar equivalents of the phenolphthalein (II).

The quenched reaction can be extracted with an aminoaryl compound of formula (IV)

(R⁴)(R⁵)N-Ar(R⁶)ₛ (IV)

wherein each R⁴ and R⁵ is independently a hydrogen or C₁₋₂₅ hydrocarbyl, each R⁶ can be independently a halogen, nitro, cyano, or C₁₋₂₅ hydrocarbyl, and s is 0-4. For example, each R⁴ and R⁵ can be independently a hydrogen or C₁₋₆ alkyl, each R⁶ can be independently a C₁₋₆ alkyl, and s is 0 or 1. For example, each R⁴ and R⁵ can be independently a hydrogen, each R⁶ can be a C₁₋₃ alkyl, and s can be 0. For example, each R⁴ and R⁵ can be a hydrogen, each R⁶ is independently a C₁₋₃ alkyl, and s is 0 or 1. For example, each R⁴ and R⁵ can be independently a hydrogen or C₁₋₆ alkyl, or each R⁴ and R⁵ is a hydrogen. For example, each R⁶ can be independently a C₁₋₆ alkyl, or each R⁶ can be independently a C₁₋₃ alkyl. In aspects, s is 0 or 1, for example, s can be 0. In some aspects, the aminoaryl compound (IV) and the primary arylamine (III) are the same, for example each can be aniline.

Extraction of the quenched reaction mixture with the aminoaryl compound (IV) provides an organic layer and an extracted aqueous layer including a crude 2-aryl-3,3-bis(hydroxyaryl)phthalimidine compound. Residual aminoaryl compound (IV) optionally can be removed from the extracted aqueous layer by azeotropic distillation or extraction with ethylene dichloride. In some aspects, the method for purification can further include precipitating the crude 2-aryl-3,3-bis(hydroxyaryl)phthalimidine compound from the extracted aqueous layer.

The extracted aqueous layer is then contacted with an adsorbent, such as activated carbon, to remove trace impurities and decolorize the layer, to form a semi-purified 2-aryl-3,3-bis(hydroxyaryl)phthalimidine compound. In some aspects, the extracted solution can be repeatedly contacted with carbon to provide the semi-purified 2-aryl-3,3-bis(hydroxyaryl)phthalimidine compound.

The semi-purified 2-aryl-3,3-bis(hydroxyaryl)phthalimidine compound is then mixed with a solution comprising an alcohol and an acid to form a purified 2-aryl-3,3-bis(hydroxyaryl)phthalimidine compound of formula (I). Exemplary alcohols include C₁₋₆ alcohols such as methanol, ethanol, isopropanol, or the like. The acid can be a mineral acid such as hydrochloric acid, sulfuric acid, nitric acid, or the like. In particular aspects, the semi-purified 2-aryl-3,3-bis(hydroxyaryl)phthalimidine compound can be mixed with a solution including methanol and hydrochloric acid to form the purified phthalimidine compound.

The semi-purified 2-aryl-3,3-bis(hydroxyaryl)phthalimidine compound can be first mixed with an alcohol, for example methanol, then mixed with acid, for example hydrochloric acid, to form the purified 2-aryl-3,3-bis(hydroxyaryl)phthalimidine of formula (I). Alternatively, the semi-purified 2-aryl-3,3-bis(hydroxyaryl)phthalimidine compound can be mixed with an alcohol, the aqueous alkali solution, and an acid to form the purified 2-aryl-3,3-bis(hydroxyaryl)phthalimidine of formula (I).

The purification or steps thereof can be performed continuously, as will be appreciated by one of ordinary skill in the art. For example, the quenching, contacting, and mixing can be a continuous process.

The method for purification can further include crystallizing the purified 2-aryl-3,3-bis(hydroxyaryl)phthalimidine of formula (I). A slurry including the purified 2-aryl-3,3-bis(hydroxyaryl)phthalimidine compound can be concentrated to provide a concentrated slurry (e.g., 60 to 80% solids), followed by separating a wet solid comprising the purified 2-aryl-3,3-bis(hydroxyaryl)phthalimidine compound from the concentrated slurry. Optionally, the wet solid can be re-slurried (e.g., 12 to 30% solids), for example a 90:10 methanol:water solution, refluxed at the boiling point of the solvent and cooled for example to -20°C, or 5 to 10°C to further precipitate the purified 2-aryl-3,3-bis(hydroxyaryl)phthalimidine compound. The wet solid can then be isolated, for example by crystallization, and the crystals are separated from the mother liquor, for example by filtration, and optionally dried to provide the purified 2-aryl-3,3-bis(hydroxyaryl)phthalimidine compound.

The mean particle size of the purified 2-aryl-3,3-bis(hydroxyaryl)phthalimidine compound can be from 200 to 400 micrometers (µm). The D(10) particle size of the purified phthalimidine compound can be 75 to 150 µm. The D(50) particle size of the purified phthalimidine compound can be 200 to 400 µm, preferably 300 to 400 µm, more preferably 300 to 350 µm. The D(90) particle size of the purified phthalimidine can be 500 to 800 µm, preferably 500 to 600 µm. In some aspects, purified phthalimide compound has a D(10) particle size of 75 to 150 µm, a D(50) particle size of 200 to 400 µm, and a D(90) particle size of 500 to 800 µm. The particle sizes reported as D(10), D(50), and D(90) are the particle size dimension where 10%, 50%, and 90% by volume, respectively, of the particles in the sample have a diameter equal to or less than the presented value, as determined in accordance with ISO 9276-2 (2014). The mean particle size can be measured using a laser particle size analyzer (LPSA).

The purified 2-aryl-3,3-bis(hydroxyaryl)phthalimidine compound of formula (I) can have a purity of greater than 99.8 wt%, preferably greater than 99.9 wt%, as determined by high performance liquid chromatography (HPLC). For example, the purified 2-aryl-3,3-bis(hydroxyaryl)phthalimidine of formula (I) can be PPPBP having a purity of greater than 99.8 wt%, preferably greater than 99.9 wt%. The purified phthalimidine compound can include less than 500 parts per million by weight (ppm) of the phenolphthalein compound (II). The purified phthalimidine compound can have an APHA color of less than 40. The purified phthalimidine compound can have less than 0.01 wt%, preferably less than 0.005 wt% of an aminophenol, based on the total weight of the purified compound, for example an aminophenol of formula (V) 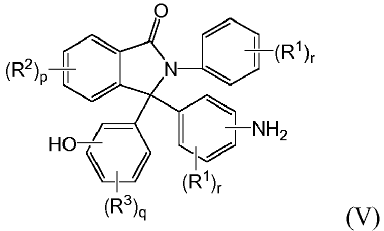 where R¹, R², R³, p, q, and r are as defined above. In some aspects, the purified phthalimidine compound can have less than 1%, preferably less than 0.15% by weight of total impurities.

Also provided herein is a 2-aryl-3,3-bis(4-hydroxyaryl) phthalimidine composition, comprising a phthalimidine of formula (I) 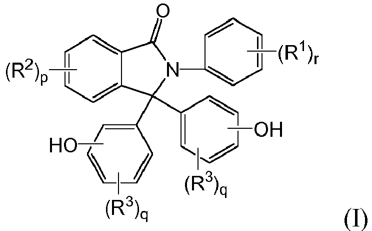 wherein R¹, R², and R³ and r, p, and q are as described above in formula (I); and wherein the composition comprises 0 to 1 wt%, preferably 0 to 0.05 wt%, more preferably 0 to 0.03 wt% of a combined aminophenol and phenolphthalein impurity, based on the composition total weight.

A polymer comprising structural units derived from the purified 2-aryl-3,3-bis(4-hydroxyaryl) phthalimidine compound manufactured by the methods described herein is also provided. The polymer can be, for example, a polycarbonate or copolycarbonate. The polymer can be a copolycarbonate comprising units derived from the purified 2-aryl-3,3-bis(4-hydroxyaryl) phthalimidine compound and units derived from bisphenol A. A method for the manufacture of a polycarbonate includes manufacturing the purified 2-aryl-3,3-bis(4-hydroxyaryl) phthalimidine compound as described herein; and polymerizing the purified 2-aryl-3,3-bis(4-hydroxyaryl) phthalimidine compound in the presence of a carbonate source.

The polycarbonate or copolycarbonate comprising structural units derived from the purified 2-aryl-3,3-bis(4-hydroxyaryl) phthalimidine compound can have 0 to 0.2 wt%, preferably 0 to 0.1 wt%, more preferably 0 to 0.025 wt% of a combined aminophenol and phenolphthalein impurity, based on the total polycarbonate weight.

The purified 2-aryl-3,3-bis(4-hydroxyaryl)phthalimidine compounds, including the exemplary purified 2-phenyl-3,3-bis(4-hydroxyphenyl)phthalimidine (PPPBP), are commercially valuable monomers and comonomers for producing a variety of polymers and copolymers formed by reactions of the phenolic OH groups of the purified 2-aryl-3,3-bis(4-hydroxyaryl)phthalimidines. Exemplary polymers that can be produced include homopolymers and copolymers of a polycarbonate, a polyestercarbonate, a polyester, a polyesteramide, a polyimide, a polyetherimide, a polyamideimide, a polyether, a polyethersulfone, a polycarbonate-polyorganosiloxane block copolymer, a copolymer comprising aromatic ester, ester carbonate, and carbonate repeat units, and a polyetherketone. An example of a copolymer comprising aromatic ester, estercarbonate, and carbonate repeat units is the copolymer produced by the reaction of a hydroxy-terminated polyester, such as the product of reaction of isophthaloyl chloride and terephthaloyl chloride with resorcinol, with phosgene and an aromatic dihydroxy compound, such as bisphenol A.

Polycarbonates having low color properties can be synthesized, wherein the polycarbonates include structural units of formula (VI) 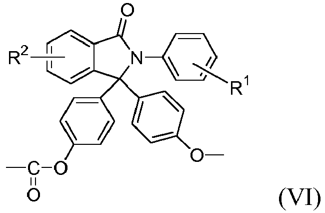 that are derived from the purified 2-aryl-3,3-bis(4-hydroxyaryl)phthalimidine compound, wherein R¹ and R² are as described previously; and the C=O structural units are derived from a C=O donor such as a carbonic acid diester in a melt transesterification process, or phosgene in an interfacial process.

Specific polycarbonates are copolycarbonates having structural units derived from the purified phthalimidine compound and a dihydroxy compound of the formula HO-R¹-OH, in particular of formula (VII)

HO-A¹-Y¹-A²-OH (VII)

wherein each of A¹ and A² is a monocyclic divalent aromatic group and Y¹ is a single bond or a bridging group having one or more atoms that separate A¹ from A². In some aspects, one atom separates A¹ from A². Specifically, each R¹ can be derived from a dihydroxy aromatic compound of formula (VIII) 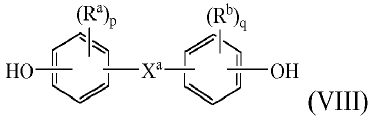 wherein R^{a} and R^{b} each represent a halogen or C₁₋₁₂ alkyl group and can be the same or different; and p and q are each independently integers of 0 to 4. X^{a} represents a single bond or a bridging group connecting the two hydroxy-substituted aromatic groups, where the single bond or the bridging group and the hydroxy substituent of each C₆ arylene group are disposed ortho, meta, or para (specifically para) to each other on the C₆ arylene group. For example, the bridging group X^{a} can be -O-, -S-, -S(O)-, -S(O)₂-, -C(O)-, or a C₁₋₁₈ organic group. The C₁₋₁₈ organic group can be cyclic or acyclic, aromatic or non-aromatic, and can further comprise heteroatoms such as halogens, oxygen, nitrogen, sulfur, silicon, or phosphorous. The C₁₋₁₈ organic group can be disposed such that the C₆ arylene groups connected thereto are each connected to a common alkylidene carbon or to different carbons of the C₁₋₁₈ organic group. For example, p and q are each 1, and R^{a} and R^{b} are each a C₁₋₃ alkyl group, specifically methyl, disposed meta to the hydroxy group on each arylene group.

Exemplary groups X^{a} include a substituted or unsubstituted C₃₋₁₈ cycloalkylidene, a C₁₋₂₅ alkylidene of formula -C(R^{c})(R^{d}) - wherein R^{c} and R^{d} are each independently hydrogen, C₁₋₁₂ alkyl, C₁₋₁₂ cycloalkyl, C₇₋₁₂ arylalkyl, C₁₋₁₂ heteroalkyl, or cyclic C₇₋₁₂ heteroarylalkyl, or a group of the formula -C(=R^{e})- wherein R^{e} is a divalent C₁₋₁₂ hydrocarbon group. Exemplary groups of this type include methylene, cyclohexylmethylene, ethylidene, neopentylidene, and isopropylidene, as well as 2-[2.2.1]-bicycloheptylidene, cyclohexylidene, cyclopentylidene, cyclododecylidene, and adamantylidene. For example, X^{a} can be a C₁₋₁₈ alkylene group, a C₃₋₁₈ cycloalkylene group, a fused C₆₋₁₈ cycloalkylene group, or a group of the formula -B¹-W-B²- wherein B¹ and B² are the same or different C₁₋₆ alkylene group and W is a C₃₋₁₂ cycloalkylidene group or a C₆₋₁₆ arylene group.

Other exemplary aromatic dihydroxy compounds of the formula HO-R¹-OH include compounds of formula (IX) 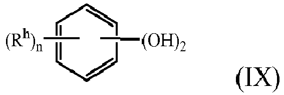 wherein each R^{h} is independently a halogen atom, a C₁₋₁₀ hydrocarbyl such as a C₁₋₁₀ alkyl group, a halogen-substituted C₁₋₁₀ alkyl group, a C₆₋₁₀ aryl group, or a halogen-substituted C₆₋₁₀ aryl group, and n is 0 to 4. For example, the halogen can be bromine.

Exemplary aromatic dihydroxy compounds include 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1-naphthylmethane, 1,2-bis(4-hydroxyphenyl)ethane, 1,1-bis(4-hydroxyphenyl)-1-phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis(hydroxyphenyl)cyclopentane, 1,1-bis(4-hydroxyphenyl)cyclohexane, 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantane, alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'- tetramethylspiro(bis)indane ("spirobiindane bisphenol"), 3,3-bis(4-hydroxyphenyl)phthalimide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, and 2,7-dihydroxycarbazole, resorcinol, substituted resorcinol compounds such as 5-methyl resorcinol, 5-ethyl resorcinol, 5-propyl resorcinol, 5-butyl resorcinol, 5-t-butyl resorcinol, 5-phenyl resorcinol, 5-cumyl resorcinol, 2,4,5,6-tetrafluoro resorcinol, 2,4,5,6-tetrabromo resorcinol, or the like; catechol; hydroquinone; substituted hydroquinones such as 2-methyl hydroquinone, 2-ethyl hydroquinone, 2-propyl hydroquinone, 2-butyl hydroquinone, 2-t-butyl hydroquinone, 2-phenyl hydroquinone, 2-cumyl hydroquinone, 2,3,5,6-tetramethyl hydroquinone, 2,3,5,6-tetra-t-butyl hydroquinone, 2,3,5,6-tetrafluoro hydroquinone, 2,3,5,6-tetrabromo hydroquinone, or the like, or a combination thereof.

Specific examples of bisphenol compounds of formula (VII) include 1,1-bis(4-hydroxyphenyl)methane, 1,1-bis(4-hydroxyphenyl)ethane, 2,2-bis(4-hydroxyphenyl)propane (hereinafter "bisphenol A" or "BPA"), 2,2-bis(4-hydroxyphenyl)butane, 2,2-bis(4-hydroxyphenyl)octane, 1,1-bis(4-hydroxyphenyl)propane, 1,1-bis(4-hydroxyphenyl) n-butane, 2,2-bis(4-hydroxy-2-methylphenyl)propane, 1,1-bis(4-hydroxy-t-butylphenyl)propane, 3,3-bis(4-hydroxyphenyl)phthalimidine, and 1,1-bis(4-hydroxy-3-methylphenyl)cyclohexane (DMBPC). Combinations comprising at least one of the foregoing dihydroxy compounds can also be used. In one specific embodiment, the polycarbonate is a linear homopolymer derived from bisphenol A, in which each of A¹ and A² is p-phenylene and Y¹ is isopropylidene.

Exemplary carbonic acid diesters for the formation of the polycarbonates in a melt transesterification process are of formula (X)

(ZO)₂C=O (X)

wherein each Z is independently an unsubstituted or substituted C₁₋₁₂ alkyl radical, or an unsubstituted or substituted C₆₋₂₂ aryl radical. Examples of carbonic acid diesters include ditolyl carbonate, m-cresyl carbonate, dinaphthyl carbonate, diphenyl carbonate, diethyl carbonate, dimethyl carbonate, dibutyl carbonate, dicyclohexyl carbonate, and combinations thereof. Use of activated aromatic carbonates that are more reactive than diphenyl carbonate is also contemplated. Exemplary activated aromatic carbonates include bis(o-methoxycarbonylphenyl)carbonate, bis(o-chlorophenyl)carbonate, bis(o-nitrophenyl)carbonate, bis(o-acetylphenyl)carbonate, bis(o-phenylketonephenyl)carbonate, bis(o-formylphenyl)carbonate. Exemplary ester-substituted diaryl carbonates include bis(methylsalicyl)carbonate (BMSC), bis(ethyl salicyl)carbonate, bis(propyl salicyl) carbonate, bis(butylsalicyl) carbonate, bis(benzyl salicyl)carbonate, bis(methyl 4-chlorosalicyl)carbonate, and the like. For example, BMSC can be used in the melt transesterification process.

The melt transesterification process is carried out by combining a catalyst, the carbonic acid diester of formula (X), the phthalimidine compound of formula (I), and optionally a dihydroxy comonomer; and mixing the reaction mixture under reactive conditions for a time period effective to produce the polycarbonate product. Exemplary melt transesterification catalysts include alkali metal compounds, alkaline earth metal compounds, tetraorganoammonium compounds, tetraorganophosphonium compounds, and combinations comprising at least one of the foregoing catalysts. Specific examples of alkali metal compounds or alkaline earth metal compounds include sodium hydroxide, potassium hydroxide, calcium hydroxide, magnesium hydroxide, sodium bicarbonate, potassium bicarbonate, sodium carbonate, potassium carbonate, lithium carbonate, sodium acetate, potassium acetate, sodium stearate, potassium stearate, sodium hydroxyborate, sodium phenoxyborate, sodium benzoate, potassium benzoate, lithium benzoate, disodium hydrogen phosphate, dipotassium hydrogen phosphate, dilithium hydrogen phosphate, disodium salts, dipotassium salts, and dilithium salts of bisphenol A, and sodium salts, potassium salts, lithium salts of phenol, or the like. Specific examples of tetraorganoammonium compounds and tetraorganophosphonium compounds include tetramethylammonium hydroxide, tetrabutylammonium hydroxide, tetraethylphosphonium hydroxide, tetrabutylphosphonium acetate, tetrabutylphosphonium hydroxide, or the like.

For example, the catalyst can be tetrabutylphosphonium acetate, or the catalyst can be a mixture of an alkali metal salt or alkaline earth metal salt with at least one quaternary ammonium compound, at least one quaternary phosphonium compound, or a mixture thereof. For example, the catalyst can be a mixture of sodium hydroxide and tetrabutylphosphonium acetate, or a mixture of sodium hydroxide and tetramethylammonium hydroxide. The catalyst can be the salt of a non-volatile inorganic acid, for example alkali metal salts of phosphites; alkaline earth metal salts of phosphites; alkali metal salts of phosphates; and alkaline earth metal salts of phosphates including NaH₂PO₃, NaH₂PO₄, Na₂H₂PO₃, KH₂PO₄, CsH₂PO₄, Cs₂H₂PO₄, or a mixture thereof. In some aspects, the transesterification catalyst include both the salt of a non-volatile acid and a basic co-catalyst such as an alkali metal hydroxide.

Any of the catalysts disclosed above can be used as combinations of two or more substances. Moreover, the catalyst can be added in a variety of forms. For example, the catalyst can be added as a solid as a powder, or it can be dissolved in a solvent, for example, in water or alcohol. The total catalyst composition can be 1 x 10⁻⁷ to 2 x 10⁻³ moles, preferably 1 x 10⁻⁶ to 4 x 10⁻⁴ moles, for each mole of the combination of, for example, the purified PPPBP and the aromatic dihydroxy comonomer.

The progress of the polymerization reaction can be monitored by measuring the melt viscosity or the weight average molecular weight of the reaction mixture using techniques known in the art such as gel permeation chromatography. These properties can be measured by taking discreet samples or can be measured on-line. After the desired melt viscosity or molecular weight is reached, the final polycarbonate product can be isolated from the reactor in a solid or molten form. The method of making polycarbonates as described in the preceding sections can be made in a batch or a continuous process.

The melt-polymerized polycarbonate can be prepared in an extruder in the presence of one or more catalysts. The reactants for the polymerization reaction can be fed to the extruder in powder or molten form. The reactants can be dry blended prior to addition to the extruder. The extruder can be equipped with pressure reducing devices (e.g., vents) that serve to remove the activated phenol byproduct and thus drive the polymerization reaction toward completion. The molecular weight of the polycarbonate product can be manipulated by controlling, among other factors, the feed rate of the reactants, the type of extruder, the extruder screw design, and configuration, the residence time in the extruder, the reaction temperature, and the pressure reducing techniques present on the extruder. The molecular weight of the polycarbonate product can also depend upon the structures of the reactants and the catalyst employed.

Alternatively, the polycarbonates can be prepared by an interfacial polymerization process. Although the reaction conditions for interfacial polymerization can vary, an exemplary process involves dissolving or dispersing a dihydric phenol reactant in aqueous caustic soda or potash, adding the resulting mixture to a water-immiscible solvent medium, and contacting the reactants with a carbonate precursor in the presence of a catalyst such as triethylamine or a phase transfer catalyst, under controlled pH conditions, e.g., about 8-about 12. Exemplary water immiscible solvents include methylene chloride, 1,2-dichloroethane, chlorobenzene, toluene, and the like.

Exemplary carbonate precursors for interfacial polymerization include a carbonyl halide such as carbonyl bromide or carbonyl chloride, or a haloformate such as a bishaloformates of a dihydric phenol (e.g., the bischloroformates of bisphenol A, hydroquinone, or the like) or a glycol (e.g., the bishaloformate of ethylene glycol, neopentyl glycol, polyethylene glycol, or the like). Combinations comprising at least one of the foregoing types of carbonate precursors can also be used. For example, an interfacial polymerization reaction to form carbonate linkages uses phosgene as a carbonate precursor (i.e., a phosgenation reaction).

Among the phase transfer catalysts that can be used for interfacial polymerization are tetraorganoammonium compounds and tetraorganophosphonium compounds of the formula (R₃)₄Q⁺X, wherein each R₃ is the same or different, and is a C₁₋₁₀ alkyl group; Q is a nitrogen or phosphorus atom; and X is a halogen atom or a C₁₋₈ alkoxy group or C₆₋₁₈ aryloxy group. Exemplary phase transfer catalysts include, for example, [CH₃(CH₂)₃]₄NX, [CH₃(CH₂)₃]₄PX, [CH₃(CH₂)₅]₄NX, [CH₃(CH₂)₆]₄NX, [CH₃(CH₂)₄]₄NX, CH₃[CH₃(CH₂)₃]₃NX, and CH₃[CH₃(CH₂)₂]₃NX, wherein X is Cl⁻, Br⁻, a C₁₋₈ alkoxy group or a C₆₋₁₈ aryloxy group. An effective amount of a phase transfer catalyst can be 0.1 to 10 wt% based on the weight of bisphenol in the phosgenation mixture. For example, an effective amount of phase transfer catalyst can be 0.5 to 2 wt% based on the weight of bisphenol in the phosgenation mixture.

All types of polycarbonate end groups are contemplated as being useful in the polycarbonate composition, provided that such end groups do not significantly adversely affect desired properties of the compositions. Branched polycarbonate blocks can be prepared by adding a branching agent during polymerization. A chain stopper (also referred to as a capping agent) can be included during polymerization. The chain stopper limits molecular weight growth rate, and so controls molecular weight in the polycarbonate. Exemplary chain stoppers include certain mono-phenolic compounds, mono-carboxylic acid chlorides, or mono-chloroformates.

The interfacial method described above can be suitably adapted to produce polycarbonates through the intermediate formation of 2-aryl-3,3-bis(4-hydroxyaryl)phthalimidine bischloroformate (i.e., bischloroformate polymerization method). For example, the method comprises reacting a 2-aryl-3,3-bis(4-hydroxyaryl)phthalimidine with phosgene in an organic solvent, and then reacting the bischloroformate either with a 2-aryl-3,3-bis(4-hydroxyaryl)phthalimidine, or an aromatic dihydroxy compound in the presence of an acid acceptor and an aqueous base to form the polycarbonate. The interfacial polymerization method and the bischloroformate polymerization method can be carried in a batch or a continuous mode using one or more reactor systems. To carry out the process in a continuous mode, one or more continuous reactors, such as for example, a tubular reactor can be used. In some embodiments, the continuous method comprises introducing into a tubular reactor system phosgene, at least one solvent (example, methylene chloride), at least one bisphenol, aqueous base, and optionally one or more catalysts (example, a trialkylamine) to form a flowing reaction mixture. The flowing mixture is then passed through the tubular reactor system until substantially all of the phosgene has been consumed. The resulting mixture is next treated with a mixture comprising an aqueous base, at least one endcapping agent, optionally one or more solvents, and at least one catalyst. The endcapped polycarbonate thus formed is continuously removed from the tubular reactor system.

The processes disclosed herein can advantageously be used to prepare polycarbonates, for example PPPBP homopolycarbonate and copolycarbonates, having a weight average molecular weight (Mw) of 3,000 to 150,000 Dalton (Da) and a glass transition temperature (Tg) of 80 to 300°C. The number average molecular weight (Mn) of the homopolycarbonate and copolycarbonates can be from 1,500 to 75,000 Da. Molecular weight can be determined by gel permeation chromatography (GPC) using homopolycarbonate standards, or by thermogravimetric analysis (TGA). The glass transition temperature can be determined by differential scanning calorimetry (DSC) or dynamic mechanical analysis (DMA).

Polymers comprising structural units derived from the phthalimidines, in particular PPPBP, can be used to manufacture polymer blends comprising the polymer and at least one other thermoplastic polymer. The at least one other thermoplastic polymer includes vinyl polymers, acrylic polymers, polyacrylonitrile, polystyrenes, polyolefins, polyesters, polyurethanes, polyamides, polysulfones, polyimides, polyetherimides, polyphenylene ethers, polyphenylene sulfides, polyether ketones, polyether ether ketones, ABS polymers, polyethersulfones, poly(alkenylaromatic) polymers, polybutadiene, polyacetals, polycarbonates, polyphenylene ethers, ethylene-vinyl acetate copolymers, polyvinyl acetate, liquid crystal polymers, ethylene-tetrafluoroethylene copolymer, aromatic polyesters, polyvinyl fluoride, polyvinylidene fluoride, polyvinylidene chloride, tetrafluoroethylene, polycarbonate-polyorganosiloxane block copolymers, copolymers comprising aromatic ester, estercarbonate, and carbonate repeat units, or a combination thereof.

The polymers and polymer blends described hereinabove are valuable for producing articles. For example, an article comprises a polymer comprising structural units derived from the purified 2-aryl-3,3-bis(4-hydroxyaryl)phthalimidine compound prepared by following the process described above.

Polymers, particularly polycarbonate homopolymers and copolymers comprising structural units derived from the high purity 2-aryl-3,3-bis(4-hydroxyaryl)phthalimidine compound in general, and PPPBP in particular exhibit lower visual coloration. As such, these polycarbonate polymers are useful for producing articles having a number of useful properties, including lower visual color, among others. The polycarbonate homopolymers and copolymers have high glass transition temperatures of higher than or equal to about 180°C. One of the unique properties of these polycarbonates, especially those that have glass transition temperatures of greater than or equal to about 180°C is that during melt processing they exhibit a shear-thinning behavior. That is, the polymers have the ability to flow under an applied shear. Therefore, standard melt processing equipment used for BPA polycarbonates can advantageously be used for producing articles. The polycarbonates also have high transparency, as measured by percent light transmission, of greater than or equal to about 85%.

In addition to the polymer(s), the thermoplastic compositions can include various additives ordinarily incorporated into polymer compositions of this type, with the proviso that the additive(s) are selected so as to not significantly adversely affect the desired properties of the thermoplastic composition, in particular low color. Such additives can be mixed at a suitable time during the mixing of the components for forming the composition. The additive can be soluble or non-soluble in polycarbonate. The additive composition can include an impact modifier, flow modifier, filler (e.g., a particulate polytetrafluoroethylene (PTFE), glass, carbon, mineral, or metal), reinforcing agent (e.g., glass fibers), antioxidant, heat stabilizer, light stabilizer, ultraviolet (UV) light stabilizer, UV absorbing additive, plasticizer, lubricant, release agent (such as a mold release agent), antistatic agent, anti-fog agent, antimicrobial agent, colorant (e.g., a dye or pigment), surface effect additive, radiation stabilizer, flame retardant, anti-drip agent (e.g., a PTFE-encapsulated styrene-acrylonitrile copolymer (TSAN)), or a combination comprising at least one or more of the foregoing. For example, a combination of a heat stabilizer, mold release agent, and ultraviolet light stabilizer can be used. In general, the additives are used in the amounts generally known to be effective. For example, the total amount of the additive composition (other than any impact modifier, filler, or reinforcing agent) can be 0.001 to 10.0 wt%, or 0.01 to 5 wt%, each based on the total weight of the polymer in the composition.

The methods described herein are further illustrated by the following non-limiting examples.

### EXAMPLES

The following components were used in the examples. Unless specifically indicated otherwise, the amount of each component is in weight percent (wt%) in the following examples, based on the total weight of the composition.

### Table 1.

**Table 1.**

| Component | Description | Source |
|---|---|---|
| PP | Phenolphthalein | Porus |
| PPPBP | 2-Phenyl-3,3-bis(4-hydroxyphenyl)phthalimidine | SABIC |

### Example 1A

70 kilograms (kg) of phenolphthalein containing 10 wt% water, 98.75 kg of aniline, and 22 kg of HCl (32 wt% in water) were combined in a 200 liter (L) glasslined reactor fitted with an overhead condenser. The reaction mixture was heated at 100-120°C and passed through a wiped film evaporator to remove water as an aniline-water azeotrope. The reaction mixture was then heated at 170°C (internal temperature), and continued until the phenolphthalein concentration was measured to be about 1 wt% in the reaction mass. The reaction mass was then allowed to cool to 150-160°C. The reaction mixture was added into another reactor containing about 650 kg of NaOH (7-10 wt% in water). The reaction mixture, once mixed with the aqueous NaOH, was cooled to about 100°C. The resulting alkali solution was treated continuously at the rate of about 70 kg/hour (hr) with aniline at a flowrate of about 40 kg/hr in a structured pack extraction column to remove the aminophenol impurity. The traces of aniline in the aqueous solution were removed by treating the alkali solution with 190 kg of 1,2-dichloroethane (EDC). The aqueous portion of the layer-separated alkali solution was isolated and passed through a packed bed of activated carbon to remove the color and other impurities. The total weight (slurry) was 600 kg. The sample was collected at this point and the semi-pure PPPBP was precipitated using HCl.

The resultant semi-pure PPPBP was analyzed by high pressure liquid chromatography (HPLC) to obtain the amount of aminophenol impurity (AP), phenolphthalein (PP), PPPBP, and total impurity in weight percent (wt%).

The analysis of semi-pure PPPBP using HPLC is given in Table 2A.

**Table 2A.**

| AP (wt%) | PP (wt%) | PPPBP (wt%) | Total impurity (wt%) |
|---|---|---|---|
| 0.002 | 0.87 | 98.54 | 1.46 |

### Example 1B

70.0 kg of phenolphthalein containing 10 wt% water, 98.75 kg of aniline, and 22 kg of HCl (32 wt% in water) were combined in a 200 L glasslined reactor fitted with overhead condenser. The reaction mixture was heated at 100-120°C and passed through a wiped film evaporator to remove the water as an aniline-water azeotrope. The reaction mixture was then heated at 170°C (internal temperature). The reaction was continued until the phenolphthalein was measured to be about 1 wt% by weight in reaction mass by HPLC. A portion of this reaction mixture was taken and designated Sample 1-1.

The reaction mass was then allowed to cool to 150-160°C. The reaction mixture was added into another reactor containing about 600 kg of NaOH (7-10 wt% in water). The reaction mixture, once mixed with the aqueous NaOH, was cooled to about 100°C. The resulting alkali solution was treated continuously at the rate of 66 kg/hr with aniline at a flowrate of 53 kg/hr in a structured pack extraction column to remove aminophenol impurity. A portion of this reaction mixture was taken and designated Sample 1-2.

Samples 1-1 and 1-2 were prepared for analysis by taking 100 mL of the alkali solution, precipitating a solid using HCl, and isolating the precipitated solids by filtration and then drying. Samples 1-1 and 1-2 were analyzed by HPLC to obtain the amount of aminophenol (AP), phenolphthalein (PP), PPPBP, and total impurity in weight percent (wt%).

The HPLC analysis of Samples 1-1 and 1-2 is given in Table 2B.

**Table 2B.**

| | AP (wt%) | PP (wt%) | PPPBP (wt%) | Total impurity (wt%) |
|---|---|---|---|---|
| Sample 1-1 | 0.866 | 0.398 | 98.2 | 1.8 |
| Sample 1-2 | 0.017 | 0.668 | 98.7 | 1.3 |

### Final Product Isolation:

2 L of the carbon treated alkali solution, prior to precipitation with HCl, comprising the semi-pure PPPBP from Example 1A was used for further evaluation of direct neutralization and crystallization of the PPPBP product in Examples 2 and 3.

### Example 2

300 mL of methanol and 29 mL of HCl were mixed in a 1 L glass reactor with an anchor type agitator. 150 mL of the carbon treated alkali solution from Example 1A was added slowly into the reactor. The reaction mixture was heated at reflux (about 63°C) for one hour and then cooled and held at 25°C for 1 hour. The temperature was maintained for another 30 minutes and then cooled to 10°C. The resulting crystallized solids were filtered and washed with 100 mL of hot water. The isolated product was dried and weighed to be 9.2 grams.

The HPLC analysis of the isolated product is given in Table 3.

**Table 3.**

| AP (wt%) | PP (wt%) | PPPBP (wt%) | Total impurity (wt%) |
|---|---|---|---|
| ND^{†} | 0.033 | 99.87 | 0.13 |

| | | | |
|---|---|---|---|
| ^{†}ND is Not Detected. | | | |

### Example 3

150 mL of the carbon treated alkali solution prepared in Example 1A was purified as follows. The concentration of semi-pure PPPBP in solution was increased from 11 wt% to 16.5 wt% using vacuum evaporation at 45°C and removing water. 300 mL of methanol and 29 mL of HCl were mixed in a 1 L glass reactor with an anchor type agitator. 100 mL of concentrated carbon treated alkali solution was added slowly to the reactor. The reaction mixture was heated at reflux (about 63°C) for one hour and then cooled to 10°C and held for 30 minutes. The resulting crystallized solids were filtered and washed with 100 mL hot water. The isolated product was dried and weighed to be 9.2 grams.

The HPLC analysis of the final product is given in Table 4.

**Table 4.**

| AP (wt%) | PP (wt%) | PPPBP (wt%) | Total impurity (wt%) |
|---|---|---|---|
| 0.0008 | 0.027 | 99.737 | 0.263 |

### Example 4

400 mL of the alkali solution prepared in Example 1B (Sample 1-2) was treated with 100 mL of EDC to remove traces of aniline. The resulting alkali solution was then treated with 10% (w/w PPPBP) special grade carbon for 1 hr.

### Example 4A

100 mL of the carbon treated solution from Example 4 was added to a mixture of 300 mL methanol and 23 mL of HCl (35 wt% it water) at 45°C. After addition was complete, the mixture was refluxed at 67°C for 1 hour and then cooled to 25°C, and then further cooled to 10°C. The resulting slurry was filtered to isolate a solid. The isolated solids were washed with hot water and analyzed for purity.

The HPLC analysis of the final product is given in Table 5A.

**Table 5A.**

| AP (wt%) | PP (wt%) | PPPBP (wt%) | Total impurity (wt%) |
|---|---|---|---|
| 0.0004 | 0.0094 | 99.92 | 0.08 |

### Example 4B

100 mL of the carbon treated solution from Example 4 was mixed with 300 mL of methanol to form a pink homogeneous liquid. HCl (35 wt% in water) was added dropwise to the pink homogeneous liquid until the pink color just disappears. The mixture was cooled to 10°C. The resulting slurry was filtered to isolate a solid. The isolated solids were washed with hot water and analyzed for purity.

The HPLC analysis of the final product is given in Table 5B.

**Table 5B.**

| AP (wt%) | PP (wt%) | PPPBP (wt%) | Total impurity (wt%) |
|---|---|---|---|
| 0.0013 | 0.0189 | 99.9 | 0.1 |

### Example 5

70.0 kg of phenolphthalein containing 10 wt% water, 98.75 kg of aniline, and 22 kg of HCl (32 wt% in water) were combined in a 200 L glass-lined reactor fitted with an overhead condenser. The reaction mixture was heated at 100-120°C and passed through a wiped film evaporator to remove water as an aniline-water azeotrope. The reaction mixture was then heated at 170°C (internal temperature). The reaction was continued until the phenolphthalein concentration was measured to be about 1 wt% of the reaction mass by HPLC. A portion of this intermediate product was removed and designated as Sample 5-1.

The reaction mass was then allowed to cool to 150-160°C. The reaction mixture was added into another reactor containing about 200 kg of NaOH (14-16 wt% in water). The reaction mixture, once mixed with the aqueous NaOH, was cooled to about 100°C. The resulting alkali solution was treated continuously at the rate of 50 kg/hr with aniline at a flowrate of 70 kg/hr in a structured pack extraction column to remove the aminophenol impurity. A portion of this intermediate product was removed and designated as Sample 5-2.

The traces of aniline in the resulting alkali solution were removed by treating the alkali solution with 110 kg of EDC. The alkali layer was separated and passed through a packed bed of activated carbon to remove the color and other impurities. A portion of this intermediate product was removed and designated as Sample 5-3.

300 L of the resulting alkali solution was added over the course of 2 to 3 hours to a mixture of 53 L of HCl and 650 L of methanol at about 40°C. After the addition was completed the mixture was refluxed at 60°C for 1 hr and then cooled to room temperature, wherein a majority of the liquid phase was drained. The resulting slurry was filtered, washed with hot water, and dried. A portion of this product was removed and designated as Sample 5-4.

The products from Samples 5-1 to 5-3 were precipitated with HCl and then isolated by filtration and drying. Sample 5-4 was used without further processing.

The HPLC analysis for Samples 5-1 to 5-4 are given in Table 6.

**Table 6.**

| | AP (wt%) | PP (wt%) | PPPBP (wt%) | Total impurity (wt%) |
|---|---|---|---|---|
| Sample 5-1 | 0.866 | 0.398 | 98.2 | 1.8 |
| Sample 5-2 | 0.017 | 0.668 | 98.7 | 1.3 |
| Sample 5-3 | 0.003 | 0.461 | 99.2 | 0.8 |
| Sample 5-4 | 0.001 | 0.016 | 99.9 | 0.1 |

### Example 6

Particle size distribution was measured using a Horiba Particle Size Analyzer, Model LA-960 Laser Particle Size Analyzer (LPSA) equipped with a fraction cell holder. The particle sizes reported as D(10), D(50), and D(90) are the particle size dimension where 10%, 50%, and 90% by volume, respectively, of the particles in the sample have a diameter equal to or less than the presented value, as determined in accordance with ISO 9276-2 (2014).

The comparative PPPBP compound was purified as follows. One part by weight of PPPBP was mixed with 4 parts by volume of a solvent system comprising water and isopropanol at ambient temperature. The resulting mixture was stirred at 95°C for two hours. Then the resulting slurry was filtered, washed with water, and dried.

FIG. 1 is a graph of volume percent (%) versus particle size (µm) showing the particle size distributions for the comparative PPPBP compound and Sample 5-4 of Example 5. The mean, D(10), D(50), and D(90) particle sizes from the comparative PPPBP and Sample 5-4 of Example 5 are shown in Table 7.

**Table 7.**

| | Comparative PPPBP | Example 5 |
|---|---|---|
| Mean | 110 | 339 |
| D(10), µm | 13 | 107 |
| D(50), µm | 97 | 322 |
| D(90), µm | 225 | 579 |

As shown in Table 7, larger particle sizes were obtained by the methods used to prepare Example 5 as compared to comparative PPPBP compound, which was prepared without using the methods as disclosed herein.

The methods and polymers are further illustrated by the following aspects, which are non-limiting.
Aspect 1: A method for the purification of a 2-aryl-3,3-bis(hydroxyaryl)phthalimidine of formula (I), the method comprising heating a reaction mixture comprising a phenolphthalein compound of formula (II) and a primary arylamine of formula (III) in the presence of an acid catalyst to form a reaction mixture; removing water from the reaction mixture, preferably by removing an arylamine-water azeotrope from the reaction mixture; quenching the reaction mixture with an aqueous alkali solution to form a quenched reaction mixture; extracting the quenched reaction mixture with an aminoaryl compound of formula (IV) to form an organic layer and an extracted aqueous layer comprising a crude 2-aryl-3,3-bis(hydroxyaryl)phthalimidine compound; contacting the extracted aqueous layer with carbon to form a semi-purified 2-aryl-3,3-bis(hydroxyaryl)phthalimidine compound, and optionally further comprising repeating the contacting with the carbon; and mixing the semi-purified 2-aryl-3,3-bis(hydroxyaryl)phthalimidine compound with a solution comprising an alcohol and an acid to form a purified 2-aryl-3,3-bis(hydroxyaryl)phthalimidine of formula (I); wherein in formulas (I), (II), (III), and (IV), each occurrence of R¹ is independently a phenyl or a C₁₋₂₅ hydrocarbyl, preferably a phenyl or a C₁₋₆ alkyl, more preferably a C₁₋₃ alkyl, each occurrence of R² and R³ is independently a C₁₋₂₅ hydrocarbyl or halogen, preferably a C₁₋₆ alkyl, more preferably a C₁₋₃ alkyl, each R⁴ and R⁵ is independently a hydrogen or C₁₋₂₅ hydrocarbyl, preferably a hydrogen or C₁₋₆ alkyl, more preferably a hydrogen, Ar is a C₆₋₁₂ aromatic ring optionally comprising up to three heteroatoms in the ring, preferably a C₆ or a C₁₂ aromatic ring, each R⁶ is independently a halogen, nitro, cyano, or C₁₋₂₅ hydrocarbyl, preferably a C₁₋₆ alkyl, more preferably a C₁₋₃ alkyl, and r, p, q, and s are each independently 0 to 4, more preferably 0 or 1, preferably 0.
Aspect 2: The method of aspect 1, wherein the semi-purified 2-aryl-3,3-bis(hydroxyaryl)phthalimidine compound is mixed with methanol, then mixed with acid to form the purified 2-aryl-3,3-bis(hydroxyaryl)phthalimidine of formula (I), or wherein the semi-purified 2-aryl-3,3-bis(hydroxyaryl)phthalimidine compound is mixed with methanol, the aqueous alkali solution, and acid to form the purified 2-aryl-3,3-bis(hydroxyaryl)phthalimidine of formula (I).
Aspect 3: The method of any one or more of the preceding aspects, wherein the quenching, contacting, and mixing is a continuous process.
Aspect 4: The method of any one or more of the preceding aspects, further comprising crystallizing the purified 2-aryl-3,3-bis(hydroxyaryl)phthalimidine of formula (I).
Aspect 5: The method of any one or more of the preceding aspects, wherein the mean particle size of the purified 2-aryl-3,3-bis(hydroxyaryl)phthalimidine of formula (I) is 300-400 micrometers, or wherein the D(10) particle size of the product is 75-150 micrometers; or the D(50) particle size of the product is 200-400 micrometers; or the D(90) particle size of the product is 500-800 micrometers.
Aspect 6: The method of any one or more of the preceding aspects, further comprising: extracting the extracted aqueous layer with an organic solvent, preferably 1,2-dichloroethane to remove aniline, or precipitating the crude 2-aryl-3,3-bis(hydroxyaryl)phthalimidine compound from the extracted aqueous layer.
Aspect 7: The method of any one or more of the preceding aspects, wherein the purified 2-aryl-3,3-bis(hydroxyaryl)phthalimidine of formula (I) comprises less than 0.01 weight percent, preferably less than 0.005 weight percent of an aminophenol, or less than 0.15 weight percent of total impurities.
Aspect 8: The method of any one or more of the preceding aspects, wherein the primary arylamine is aniline, or the acid catalyst is a mineral acid, preferably hydrochloric acid, or the aqueous alkali solution comprises an aqueous solution of an alkali metal hydroxide or an alkaline earth metal hydroxide, preferably sodium hydroxide.
Aspect 9: The method of any one or more of the preceding aspects, wherein the acid catalyst is present at a concentration of 0.5-1.5 molar equivalents of the phenolphthalein compound of formula (II), and the aqueous alkali solution is present at a concentration of 1.5-3.0 molar equivalents of the phenolphthalein compound of formula (II).
Aspect 10: The method of any one or more of the preceding aspects, wherein the aminoaryl compound of formula (IV) has the same structure as the primary arylamine of formula (III), preferably wherein the aminoaryl compound of formula (IV) is aniline.
Aspect 11: The method of any one or more of the preceding aspects, wherein the 2-aryl-3,3-bis(hydroxyaryl)phthalimidine of formula (I) is of formula (IA) wherein R¹ is a C₁₋₃ alkyl, R² is a C₁₋₃ alkyl or a halogen, p is 0 or 1, and r is 0 or 1; preferably wherein each of p and r is zero, and the 2-aryl-3,3-bis(hydroxyaryl)phthalimidine of formula (I) is 2-phenyl-3,3-bis(4-hydroxyphenyl)-2-phthalimidine.
Aspect 12: The method of any one or more of the preceding aspects, wherein the acid catalyst is hydrochloric acid, the primary arylamine is aniline, the phenolphthalein compound is phenolphthalein, and the aminoaryl compound is aniline.
Aspect 13: The method of any one or more of the preceding aspects, further comprising polymerizing the purified 2-aryl-3,3-bis(4-hydroxyaryl)phthalimidine of formula (I) in the presence of a carbonate source to manufacture a polycarbonate.
Aspect 14: A polycarbonate composition comprising the polycarbonate of aspect 13, wherein a total content of aminophenol impurity and phenolphthalein impurity is less than 0.2 weight percent, preferably less than 0.1 weight percent, more preferably less than 0.025 weight percent, based on the total weight of the polycarbonate.
Aspect 15: A 2-aryl-3,3-bis(4-hydroxyaryl)phthalimidine composition, comprising the purified 2-aryl-3,3-bis(hydroxyaryl)phthalimidine of any one or more of aspects 1 to 12, wherein a total content of aminophenol impurity and phenolphthalein impurity is less than 1 weight percent, preferably less than 0.05 weight percent, more preferably less than 0.03 weight percent, based on the total weight of the composition.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

The endpoints of all ranges directed to the same component or property are inclusive and independently combinable (e.g., ranges of "less than or equal to 25 wt%, or 5-20 wt%," is inclusive of the endpoints and all intermediate values of the ranges of "5 wt% to 25 wt%," etc.). Disclosure of a narrower range or more specific group in addition to a broader range is not a disclaimer of the broader range or larger group. The terms "first," "second," and the like, do not denote any order, quantity, or importance, but rather are used to distinguish one element from another. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. "Combination thereof' is an open ended term that includes one or more of the listed items and can optionally include similar non-listed items. Reference throughout the specification to "some aspects", "an aspect", and so forth, means that a particular element described for the aspect is included in at least one aspect described herein, and may or may not be present in other aspects. In addition, it is to be understood that the described elements in any aspect may be combined with other elements of any aspect in any suitable manner in the various aspects

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this application belongs. All cited patents, patent applications, and other references are incorporated herein by reference in their entirety. However, if a term in the present application contradicts or conflicts with a term in the incorporated reference, the term from the present application takes precedence over the conflicting term from the incorporated reference.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group.

The term "hydrocarbyl" is defined herein as a monovalent moiety formed by removing a hydrogen atom from a hydrocarbon. The term "hydrocarbon" means any compound or group comprising carbon and hydrogen. The term "alkyl" means a branched or straight chain, unsaturated aliphatic hydrocarbon group, e.g., methyl, ethyl, n-propyl, i-propyl, n-butyl, s-butyl, t-butyl, n-pentyl, s-pentyl, and n- and s-hexyl. "Alkenyl" means a straight or branched chain, monovalent hydrocarbon group having at least one carbon-carbon double bond (e.g., ethenyl (-HC=CH₂)). "Alkoxy" means an alkyl group that is linked via an oxygen (i.e., alkyl-O-), for example methoxy, ethoxy, and sec-butyloxy groups. "Alkylene" means a straight or branched chain, saturated, divalent aliphatic hydrocarbon group (e.g., methylene (-CH₂-) or, propylene (-(CH₂)₃-)). "Cycloalkylene" means a divalent cyclic alkylene group, -CₙH₂ₙ₋ₓ, wherein x is the number of hydrogens replaced by cyclization(s). "Cycloalkenyl" means a monovalent group having one or more rings and one or more carbon-carbon double bonds in the ring, wherein all ring members are carbon (e.g., cyclopentyl and cyclohexyl). "Aryl" means an aromatic hydrocarbon group containing the specified number of carbon atoms, such as phenyl, tropone, indanyl, or naphthyl. "Arylene" means a divalent aryl group. "Alkylarylene" means an arylene group substituted with an alkyl group. "Arylalkylene" means an alkylene group substituted with an aryl group (e.g., benzyl). The prefix "halo" means a group or compound including one more of a fluoro, chloro, bromo, or iodo substituent. A combination of different halo groups (e.g., bromo and fluoro), or only chloro groups can be present. The prefix "hetero" means that the compound or group includes at least one ring member that is a heteroatom, wherein the heteroatom(s) is each independently N, O, S, Si, or P.

Unless substituents are otherwise specifically indicated, each of the foregoing groups can be unsubstituted or substituted, provided that the substituted atom's normal valence is not exceeded and the substitution does not significantly adversely affect synthesis, stability, or use of the compound. "Substituted" means that the compound, group, or atom is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g., benzyl), C₇₋₁₂ alkylarylene (e.g, toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-). The number of carbon atoms indicated in a group is exclusive of any substituents. For example -CH₂CH₂CN is a C₂ alkyl group substituted with a cyano substituent.

While particular aspects have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A method for the purification of a 2-aryl-3,3-bis(hydroxyaryl)phthalimidine compound of formula (I) 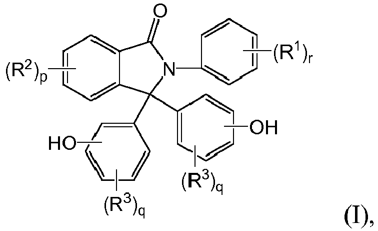 the method comprising
heating a reaction mixture comprising a phenolphthalein compound of formula (II) 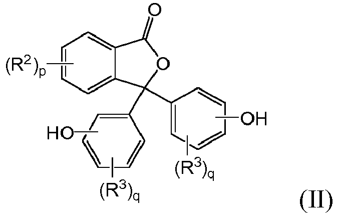 and a primary arylamine of formula (III) 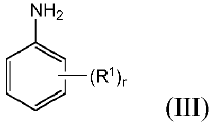 in the presence of an acid catalyst to form a reaction mixture;
removing water from the reaction mixture, preferably by removing an arylamine-water azeotrope from the reaction mixture;
quenching the reaction mixture with an aqueous alkali solution to form a quenched reaction mixture;
extracting the quenched reaction mixture with an aminoaryl compound of formula (IV)
(R⁴)(R⁵)N-Ar(R⁶)ₛ (IV)
to form an organic layer and an extracted aqueous layer comprising a crude 2-aryl-3,3-bis(hydroxyaryl)phthalimidine compound;
contacting the extracted aqueous layer with carbon to form a semi-purified 2-aryl-3,3-bis(hydroxyaryl)phthalimidine compound, and optionally further comprising repeating the contacting with the carbon; and
mixing the semi-purified 2-aryl-3,3-bis(hydroxyaryl)phthalimidine compound with a solution comprising an alcohol and an acid to form a purified 2-aryl-3,3-bis(hydroxyaryl)phthalimidine compound of formula (I);
wherein in formulas (I), (II), (III), and (IV)
each occurrence of R¹ is independently a phenyl or a C₁₋₂₅ hydrocarbyl, preferably a phenyl or a C₁₋₆ alkyl, more preferably a C₁₋₃ alkyl,
each occurrence of R² and R³ is independently a C₁₋₂₅ hydrocarbyl or halogen, preferably a C₁₋₆ alkyl, more preferably a C₁₋₃ alkyl,
each R⁴ and R⁵ is independently a hydrogen or C₁₋₂₅ hydrocarbyl, preferably a hydrogen or C₁₋₆ alkyl, more preferably a hydrogen,
Ar is a C₆₋₁₂ aromatic ring optionally comprising up to three heteroatoms in the ring, preferably a C₆ or a C₁₂ aromatic ring,
each R⁶ is independently a halogen, nitro, cyano, or C₁₋₂₅ hydrocarbyl, preferably a C₁₋₆ alkyl, more preferably a C₁₋₃ alkyl, and
r, p, q, and s are each independently 0 to 4, more preferably 0 or 1, preferably 0.

2. The method of claim 1,
wherein the semi-purified 2-aryl-3,3-bis(hydroxyaryl)phthalimidine compound is mixed with methanol, then mixed with acid to form the purified 2-aryl-3,3-bis(hydroxyaryl)phthalimidine of formula (I), or
wherein the semi-purified 2-aryl-3,3-bis(hydroxyaryl)phthalimidine compound is mixed with methanol, the aqueous alkali solution, and acid to form the purified 2-aryl-3,3-bis(hydroxyaryl)phthalimidine of formula (I).

3. The method of any one or more of the preceding claims, wherein the quenching, contacting, and mixing is a continuous process.

4. The method of any one or more of the preceding claims, further comprising crystallizing the purified 2-aryl-3,3-bis(hydroxyaryl)phthalimidine of formula (I).

5. The method of any one or more of the preceding claims, wherein
the mean particle size of the purified 2-aryl-3,3-bis(hydroxyaryl)phthalimidine of formula (I) is 300-400 micrometers, or wherein
the D(10) particle size of the product is 75-150 micrometers; or
the D(50) particle size of the product is 200-400 micrometers; or
the D(90) particle size of the product is 500-800 micrometers.

6. The method of any one or more of the preceding claims, further comprising
extracting the extracted aqueous layer with an organic solvent, preferably 1,2-dichloroethane to remove aniline, or
precipitating the crude 2-aryl-3,3-bis(hydroxyaryl)phthalimidine of formula (I) from the extracted aqueous layer.

7. The method of any one or more of the preceding claims, wherein the purified 2-aryl-3,3-bis(hydroxyaryl)phthalimidine of formula (I) comprises
less than 0.01 weight percent, preferably less than 0.005 weight percent of an aminophenol, or
less than 0.15 weight percent of total impurities.

8. The method of any one or more of the preceding claims, wherein
the primary arylamine is aniline, or
the acid catalyst is a mineral acid, preferably hydrochloric acid, or
the aqueous alkali solution comprises an aqueous solution of an alkali metal hydroxide or an alkaline earth metal hydroxide, preferably sodium hydroxide.

9. The method of any one or more of the preceding claims, wherein
the acid catalyst is present at a concentration of 0.5-1.5 molar equivalents of the phenolphthalein compound of formula (II), and
the aqueous alkali solution is present at a concentration of 1.5-3.0 molar equivalents of the phenolphthalein compound of formula (II).

10. The method of any one or more of the preceding claims,
wherein the aminoaryl compound of formula (IV) has the same structure as the primary arylamine of formula (III),
preferably wherein the aminoaryl compound of formula (IV) is aniline.

11. The method of any one or more of the preceding claims, wherein the 2-aryl-3,3-bis(hydroxyaryl)phthalimidine of formula (I) is of formula (IA) 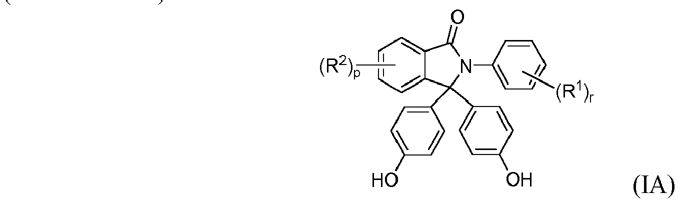 wherein R¹ is a C₁₋₃ alkyl, R² is a C₁₋₃ alkyl or a halogen, p is 0 or 1, and r is 0 or 1;
preferably wherein each of p and r is zero, and the 2-aryl-3,3-bis(hydroxyaryl)phthalimidine of formula (I) is 2-phenyl-3,3-bis(4-hydroxyphenyl)-2-phthalimidine.

12. The method of any one or more of the preceding claims, wherein the acid catalyst is hydrochloric acid, the primary arylamine is aniline, the phenolphthalein compound is phenolphthalein, and the aminoaryl compound is aniline.

13. The method of any one or more of the preceding claims, further comprising polymerizing the purified 2-aryl-3,3-bis(4-hydroxyaryl)phthalimidine compound of formula (I) in the presence of a carbonate source to manufacture a polycarbonate.

14. A polycarbonate composition comprising the polycarbonate of claim 13, wherein a total content of aminophenol impurity and phenolphthalein impurity is less than 0.2 weight percent, preferably less than 0.1 weight percent, more preferably less than 0.025 weight percent, based on the total weight of the polycarbonate.

15. A 2-aryl-3,3-bis(4-hydroxyaryl)phthalimidine composition, comprising the purified 2-aryl-3,3-bis(hydroxyaryl)phthalimidine compound of any one or more of claims 1 to 12, wherein a total content of aminophenol impurity and phenolphthalein impurity is less than 1 weight percent, preferably less than 0.05 weight percent, more preferably less than 0.03 weight percent, based on the total weight of the composition.
